# EUROPÄISCHE PATENTSCHRIFT

(11) **EP 0 834 493 B1**
(45) Veröffentlichungstag und Bekanntmachung des Hinweises auf die Patenterteilung: **09.05.2001**
(21) Anmeldenummer: 97116761.4
(22) Anmeldetag: 26.09.1997
(51) Int. Cl.: C07C 37/88, C07C 39/04, C07C 49/08

(54) **Verfahren zum Transport und zur Lagerung von Phenol**
Process for the transport and storage of phenol
Procédé pour le transport et le stockage du phénol

(30) Priorität: 02.10.1996 DE 19640710
(43) Veröffentlichungstag der Anmeldung: 08.04.1998
(73) Patentinhaber: Phenolchemie GmbH & Co. KG, 45966 Gladbeck (DE); BAYER AG, 51368 Leverkusen (DE)
(72) Erfinder: Kleinloh, Werner, Dr., 45721 Haltern (DE); Schnurr, Otto, Dr., 2950 Kapellen (BE); van Osselaer, Tony, Dr., 9100 Belsele (BE); Wulff, Claus, Dr., 47800 Krefeld (DE); Zaby, Gottfried, Dr., 51375 Leverkusen (DE)

(56) Entgegenhaltungen:
- DE-A- 3 122 799

## Beschreibung

Die Erfindung betrifft ein Verfahren zum Transport und zur Lagerung von Phenol.

Phenol ist ein Basisprodukt der Petrochemie, von dem weltweit mehr als 5 Mio. Tonnen pro Jahr erzeugt werden. Phenol dient als Ausgangsprodukt für zahlreiche Zwischen- und Fertigprodukte. Ein großer Anteil des Phenols wird zu Phenol-Formaldehyd-Harzen weiterverarbeitet. Daneben erfolgt die Herstellung von Bisphenol A (2,2'-Di-(4-hydroxyphenyl)-propan) aus Phenol und Aceton in großem Umfang, da Bisphenol A als Ausgangsprodukt für Polycarbonate und Epoxidharze zunehmende Bedeutung erzielt hat.

Phenol hat einen Schmelz- bzw. Erstarrungspunkt von 41 °C; es ist also bei den üblichen Umgebungstemperaturen fest. Phenol wird jedoch nur in unbedeutend kleinen Mengen in fester Form gehandelt. Üblicherweise wird Phenol als Flüssigkeit mit Temperaturen oberhalb seines Erstarrungspunktes transportiert und gelagert. Um das Phenol flüssig zu halten, sind die Rohrleitungen, Umschlageinrichtungen sowie die Lager- und Transportbehälter gewöhnlich gegen Wärmeverluste isoliert. In der Regel ist auch eine zusätzliche Beheizungsmöglichkeit erforderlich. Es werden also meist wärmeisolierte, beheizbare Transport- und Lagersysteme eingesetzt.

Als Behältermaterial wird im allgemeinen Stahl verwendet. Phenol neigt zur Verfärbung durch Oxidationsprodukte des Phenols. Soll die Farblosigkeit des Phenols möglichst lang erhalten bleiben, werden Systeme aus Edelstahl eingesetzt.

Bei der Lagerung und beim Transport von flüssigem Phenol ist darauf zu achten, daß die Temperatur unter 70 °C bleibt, da die untere Explosionsgrenze für Phenol-Luft-Gemische bei einer Sättigungstemperatur von ca. 73 °C erreicht wird. Bei Lagertemperaturen oberhalb des Flammpunktes muß der Gasraum im Behälter aufwendig, z. B. durch Begasung mit Stickstoff, inertisiert werden.

Eine andere bekannte Handelsform von Phenol ist ein Gemisch aus Phenol mit Wasser, wobei durch Zugabe von Wasser theoretisch ein Erstarrungspunkt des Gemisches Phenol/Wasser zwischen 0 °C und 41 °C eingestellt werden kann. In der Praxis arbeitet man jedoch nur im Konzentrationsbereich von 8 bis 10 Gew.-% Wasser in der Mischung. Gibt man mehr Wasser in das System, so zerfällt dieses in eine wasserreiche und eine phenolreiche Phase. Das System Phenol/Wasser weist unterhalb von 69 °C eine Mischungslücke auf (vgl. Ullmanns Encyklopädie der technischen Chemie, 4. Aufl., 1979, Bd. 18, S. 177). Eine Mischung von Phenol mit ca. 10 Gew.-% Wasser besitzt einen Erstarrungspunkt von ca. 13, 5 °C; eine Mischung mit 20 Gew.-% Wasser einen Erstarrungspunkt von ca. 5 °C. Die Mischungslücke des Systems Phenol/Wasser bei 10 °C liegt zwischen Phenolgehalten von 7 Gew.-% und 75 Gew.-%. Je nach klimatischen Bedingungen können beim Transport und bei der Lagerung von Phenol jedoch auch Temperaturen von 0 °C und darunter auftreten. Daher macht auch die Zugabe von Wasser zum Phenol den Einsatz von beheizbaren, wärmeisolierten Behältern nicht in jedem Fall entbehrlich. Außerdem muß das Wasser - je nach Verwendungszweck des Phenols - später in der Regel aufwendig wieder vom Phenol getrennt werden.

Aufgabe der vorliegenden Erfindung ist es daher, ein Verfahren zum Transport und zur Lagerung von Phenol in flüssiger Phase bereitzustellen, das bei herkömmlichen Transport- und Lagerbedingungen den Einsatz beheizbarer, wärmeisolierter Systeme nicht erforderlich macht.

Diese Aufgabe wird erfindungsgemäß gelöst durch ein Verfahren zum Transport und zur Lagerung von Phenol gemäß Patentanspruch 1, das dadurch gekennzeichnet ist, daß dem flüssigen Phenol bis zu 70 Gew.-% Aceton zugegeben werden. Bevorzugt beträgt der Acetongehalt in der Mischung Phenol/Aceton zwischen 20 und 60 Gew.-%, besonders bevorzugt zwischen 45 und 55 Gew.-%. Die so erhaltenen Mischungen können bei entsprechenden klimatischen Verhältnissen ohne Zusatzbeheizung und Wärmeisolation als flüssige Phase gelagert oder umgeschlagen und transportiert werden.

Das System Phenol/Aceton besitzt keine Mischungslücke, und es lassen sich ohne weiteres Mischungen mit Erstarrungspunkten unter 0 °C einstellen. Ein Gemisch aus Phenol mit 15 Gew.-% Aceton hat z. B. einen Erstarrungspunkt von ca. 20 °C; bei 50 Gew.-% Aceton ergibt sich ca. 0 °C, bei 70 Gew.-% Aceton -40 °C als Erstarrungspunkt. Phenol/Aceton-Mischungen mit 20 bis 60 Gew.-% Aceton besitzen Erstarrungspunkte zwischen +17 °C und -20 °C; bei Phenol/Aceton-Mischungen mit 45 bis 55 Gew.-% Aceton liegen die Erstarrungspunkte zwischen +5 °C und -10 °C.

Durch Zugabe von Aceton zu Phenol lassen sich damit jederzeit Mischungen einstellen, deren Erstarrungspunkt so niedrig ist, daß die Mischung bei vorgegebenen Transport- und Lagerbedingungen stets flüssig ist. Dadurch werden aufwendige beheizbare und/oder wärmeisolierte Transport- und Lagersysteme entbehrlich. Dies führt nicht nur zu erheblichen Kosteneinsparungen, sondern erhöht zusätzlich wesentlich die Flexibilität in der Logistik, da keine speziellen Tanks erforderlich sind. Da die Lagerung und der Transport von Phenol in flüssiger Phase nach dem erfindungsgemäßen Verfahren bei Umgebungstemperatur und damit bei deutlich niedrigeren Temperaturen als bisher erfolgen können, sinkt auch die Neigung des Phenols zur Verfärbung durch Oxidation mit Luftsauerstoff.

Beim Transport von reinem flüssigen Phenol besteht ferner stets die Gefahr des Einfrierens durch z. B. den Ausfall der Zusatzbeheizung oder Schäden an der Wärmeisolation, weshalb größere Transportentfernungen gemieden werden. Beim Wiederauftauen fest gewordenen Phenols kommt es außerdem sehr häufig zur Qualitätsminderung durch Verfärbung. Diese Mängel werden durch Anwendung des erfindungsgemäßen Verfahrens für den Phenoltransport sicher vermieden.

Die üblichen Umgebungstemperaturen für den Transport bzw. die Lagerung von Phenol liegen etwa zwischen -20 °C und +35 °C, so daß Phenol/Aceton-Mischungen mit einem Acetongehalt bis 70 Gew.-% ausreichen, um das Produkt stets flüssig zu halten. Dadurch wird nicht nur die Lagerung von Phenol in z. B. Tanks oder der Transport in Tankwagen, Kesselwagen, Tankschiffen usw. erleichtert, auch der Transport in Rohrleitungen über größere Entfernungen wird erleichtert und teilweise sogar erst ermöglicht. Rohrleitungstransporte von Phenol über längere Strecken sind in der Vergangenheit häufig daran gescheitert, daß die Wirtschaftlichkeit wegen der Notwendigkeit beheizter und isolierter Leitungen nicht gegeben war. Bei unterirdischer Leitungsführung muß zudem Wasser von der Isolierung ferngehalten werden, bzw. es muß eine teure, wasserundurchlässige Isolierung gewählt werden.

Beim Rohrleitungstransport von Phenol/Aceton-Gemischen kann auf die Isolierung völlig verzichtet werden. Durch Veränderung des Acetongehaltes kann zudem der Erstarrungspunkt des Gemisches kurzfristig den äußeren Bedingungen angepaßt werden. Für Unterbrechungen des Förderbetriebes kann auch ein acetonreicheres Gemisch in die Leitung gefüllt werden, womit alle extremen äußeren Bedingungen abgedeckt sind.

Das spätere Abtrennen des Acetons zur Gewinnung von reinem Phenol kann für solche Anwendungsfälle von Phenol entfallen, in denen Phenol und Aceton ohnehin gemeinsam gebraucht werden, also insbesondere z. B. bei der wirtschaftlich und vom Umfang her bedeutsamen Weiterverarbeitung von Phenol zu Bisphenol A. Vor allem für diese Anwendung bietet sich der Umschlag und Transport eines Phenol/Aceton-Gemisches geradezu an. Auch hier werden hohe Kosten- und Flexibilitätsvorteile erzielt; und das zugegebene Aceton muß zur Weiterverarbeitung nicht abgetrennt werden.

Die Tanklagerkapazität beim Empfänger/Weiterverarbeiter kann entsprechend kleiner ausgelegt werden. Dies verringert die Kapitalkosten und das Umlaufvermögen von Bisphenol A-Anlagen.

Auch just in time-Liefervereinbarungen sind einfacher zu organisieren, insbesondere dann, wenn die räumliche Entfernung zwischen Lieferant und Kunde die Verwendung einer Rohrleitungsverbindung ermöglicht.

Der Transport eines Gemisches von Phenol und Aceton bietet nicht nur Vorteile, wenn ohnehin beide Stoffe im anschließenden Produktionsbetrieb gebraucht werden.

Mit dieser Erfindung kann das Stoffgemisch in kundennahen Verteilzentren vorgehalten werden. Aceton kann aus dem Gemisch durch einfache Destillation als reiner Stoff jederzeit nach Bedarf freigesetzt werden. Desgleichen kann reines Phenol durch Abdestillieren des Acetons nach Bedarf wieder isoliert werden. So ist es möglich, kundennah beide reinen Stoffe jederzeit zur Verfügung zu haben, und gleichzeitig die anderen genannten Vorteile von Transport und Lagerung des Stoffgemisches zu nutzen.

Wird das Phenol nach dem Hock-Verfahren hergestellt, fällt Aceton als Koppelprodukt an und steht damit zur Anwendung des erfindungsgemäßen Verfahrens in in der Regel ausreichender Menge zur Verfügung. Die Anwendung des erfindungsgemäßen Verfahrens ist also besonders einfach und vorteilhaft, wenn das Phenol nach dem Hock-Verfahren gewonnen wird.

Das erfindungsgemäße Verfahren wird durch die nachfolgenden Beispiele näher erläutert, ohne darauf beschränkt zu sein:

### Beispiel 1:

### Transport von Phenol über Straße und Schiene in Mitteleuropa im Sommer

Wenn Phenol mit Aceton verdünnt wird, bis die Mischung 40 Gew.-% Aceton enthält, ergibt sich ein Erstarrungspunkt von 8 °C. Dieses Gemisch ist in den Sommermonaten in Mitteleuropa nicht einfriergefährdet. Die Umschlageinrichtungen selbst brauchen nicht beheizt und isoliert zu sein, die Transportbehälter ebenfalls nicht. Dies bringt nicht nur Kosteneinsparungen, sondern erhöht zusätzlich die Flexibilität in der Logistik.

### Beispiel 2:

### Transport von Phenol über Straße und Schiene in Osteuropa im Winter

In Osteuropa muß im Winter mit Temperaturen von -20 °C gerechnet werden. Ein Gemisch mit 60 Gew.-% Aceton erstarrt erst bei -20 °C. Insbesondere wegen der langen Transportwege bietet das Ausschalten der Einfriergefahr große Vorteile. Beim Wiederauftauen fest gewordenen Phenols kommt es außerdem sehr häufig zur Qualitätsminderung durch Verfärbung. Dieses Problem entfällt bei Anwendung des erfindungsgemäßen Verfahrens.

### Beispiel 3:

### Tanklagerung in Mitteleuropa

Wenn reines Phenol in Tanks gelagert wird, so müssen die Tanks isoliert und beheizt sein. Im entsprechenden Gemisch mit Aceton können nicht isolierte Tanks verwendet werden, was nicht nur die Kosten reduziert, sondern gleichzeitig auch Flexibilität beim Tankeinsatz gewährleistet. 55 Gew.-% Aceton im Gemisch senken den Erstarrungspunkt bereits auf -11 °C ab.

### Beispiel 4:

### Schifftransport

Wenn reines Phenol in Schiffen transportiert wird, so ist eine Isolation gegen das umgebende Wasser besonders wichtig, da die Kühlwirkung von Wasser beträchtlich größer ist als die von Luft. Transporte in nicht isolierten Schiffstanks sind einfacher zu organisieren und zusätzlich noch billiger als Transporte in isolierten und beheizbaren Schiffstanks. Um einen Erstarrungspunkt unter 0 °C zu erreichen, muß eine Mischung mit 50 Gew.-% Aceton eingestellt werden.

### Beispiel 5:

### Unterirdischer Rohrleitungstransport über 5 km

In einem konkreten Fall wurde ein kontinuierlicher Rohrleitungstransport von reinem Phenol über 5 km in Belgien gegen einen alternativen Schiffstransport untersucht. Der Rohrleitungstransport von reinem Phenol erwies sich als unwirtschaftlich. Wird statt dessen jedoch ein Phenol/Aceton-Gemisch mit mindestens 45 Gew.-% Aceton transportiert, so genügt die Verlegung einer nicht isolierten unterirdischen Leitung. Ein kontinuierlicher Rohrleitungstransport wird dadurch wirtschaftlicher als der Schiffstransport. Die errechnete Kapitalrückflußzeit liegt unter 2 Jahren.

### Beispiel 6 :

### Oberirdischer Rohrleitungstransport über 2 km

Für ein Projekt im Süden der USA wurde ein Rohrleitungstransport vom Schiffsanlegeplatz zum 1,5 km entfernten Tanklager untersucht. Die Leitung soll zum Entladen und Beladen von Schiffen benutzt werden. Die Baukosten für eine beheizte und isolierte Leitung DN 200 liegen um ca. 50 % höher als für eine nicht isolierte Leitung für ein Aceton-Phenol-Gemisch, wobei noch keine zweite Leitung für Aceton mitgerechnet wurde.

### Beispiel 7:

### Rohrleitungstransport über 20 km

Ein Pipelinetransport von reinem Phenol in einer beheizten und isolierten Leitung ist erheblich teurer als ein alternativer Transport mit Fahrzeugen oder Schiffen. Zudem wäre nur mit erheblichem Aufwand sicherzustellen, daß die Beheizung und Isolierung der Pipeline lückenlos funktionieren. Deshalb scheitert ein solches Projekt auch an technischen Problemen.

In diesem konkreten Fall wird alternativ eine nicht isolierte Leitung DN 80 für den erfindungsgemäßen Phenoltransport zugrundegelegt, die auf einem vorhandenen Unterbau verlegt wird. Wenn 50.000 jato eines Gemisches aus Phenol mit 55 Gew.-% Aceton gefördert werden, bleibt das Gemisch bis -10 °C flüssig. Der Transportkostenvorteil gegenüber Landfahrzeugen ergibt einen Kapitalrückfluß in deutlich weniger als 2 Jahren.

### Beispiel 8:

### Rückgewinnung von Phenol und Aceton aus dem Transport-/Lagergemisch

Phenol und Aceton sind sehr einfach destillativ zu trennen. In einer kontinuierlich betriebenen Destillationskolonne kann reines Aceton als Kopfprodukt und reines Phenol als Sumpfprodukt gewonnen werden. In einer diskontinuierlich betriebenen Destillationskolonne kann reines Aceton als erste Fraktion abdestilliert werden. Die zweite Fraktion enthält ein Gemisch, das zum Einspeistank zurückgefahren werden kann. Als Sumpfprodukt verbleibt reines Phenol.

Solche kombinierten Lager- und Trennanlagen können überall in regionalen Märkten zum Einsatz kommen.

## Patentansprüche

1. Verfahren zum Transport und zur Lagerung von Phenol,
dadurch gekennzeichnet,
daß dem flüssigen Phenol bis zu 70 Gew.-% Aceton zugegeben werden.

2. Verfahren nach Anspruch 1,
dadurch gekennzeichnet,
daß dem Phenol 20 bis 60 Gew.-% Aceton zugegeben werden.

3. Verfahren nach Anspruch 2,
dadurch gekennzeichnet,
daß dem Phenol 45 bis 55 Gew.-% Aceton zugegeben werden.

4. Verfahren nach zumindest einem der vorherigen Ansprüche,
dadurch gekennzeichnet,
daß die Transport- bzw. Lagerbehälter nicht beheizbar und/oder nicht wärmeisoliert ausgeführt sind.

5. Verfahren nach zumindest einem der vorherigen Ansprüche,
dadurch gekennzeichnet,
daß das Phenol nach dem Hock-Verfahren hergestellt ist.

## Claims

1. A method for the transport and storage of phenol, characterised in that up to 70% by weight of acetone is added to the liquid phenol.

2. A method according to claim 1, characterised in that 20 to 60% by weight of acetone is added to the phenol.

3. A method according to claim 2, characterised in that from 45 to 55% by weight of acetone is added to the phenol.

4. A method according to at least one of the preceding claims, characterised in that the transport or storage vessels are not constructed so as to be heatable and/or are not constructed so as to be thermally insulated.

5. A method according to at least one of the preceding claims, characterised in that the phenol is prepared by the Hock process.

## Revendications

1. Procédé de transport et de stockage de phénol,
caractérisé en ce qu'
au phénol liquide on ajoute jusqu'à 70 % en poids d'acétone.

2. Procédé selon la revendication 1,
caractérisé en ce qu'
au phénol on ajoute de 20 à 60 % en poids d'acétone.

3. Procédé selon la revendication 2,
caractérisé en ce qu'
au phénol on ajoute de 45 à 55 % en poids d'acétone.

4. Procédé selon au moins une des revendications précédentes,
caractérisé en ce que
le récipient de transport ou le récipient de stockage sont réalisés non chauffants et/ou non isolés thermiquement.

5. Procédé selon au moins une des revendications précédentes,
caractérisé en ce que
le phénol est produit selon le procédé Hock.
